(19) Europäisches Patentamt
European Patent Office
Office européen des brevets

(11) Publication number : **0 609 032 A1**

# (12) EUROPEAN PATENT APPLICATION

(21) Application number : **94300454.9**

(22) Date of filing : **21.01.94**

(51) Int. Cl.[5] : **C07C 251/24,** A61K 31/13, A61K 31/195, A61K 31/42, C07D 263/04, C07D 239/54, C07D 407/04, A61K 31/505

(30) Priority : **25.01.93 GB 9301353**
**12.02.93 GB 9302797**

(43) Date of publication of application :
**03.08.94 Bulletin 94/31**

(84) Designated Contracting States :
**AT BE CH DE DK ES FR GB GR IE IT LI LU MC NL PT SE**

(71) Applicant : **NORSK HYDRO a.s.**
**Bygdoy allé 2**
**N-0240 Oslo 2 (NO)**

(72) Inventor : **Pettersen, Erik Olai**
**Tokerudberget 10**
**N-0986 Oslo (NO)**
Inventor : **Larsen, Rolf Olaf**
**Nustadringen 15**
**N-3970 Langesund (NO)**
Inventor : **Dornish, John Michael**
**Keivveien 44B**
**N-1340 Bekkestua (NO)**
Inventor : **Borretzen, Bernt**
**Granlia 3**
**N-3940 Heistad (NO)**
Inventor : **Oftebro, Reidar**
**Johs. Hartmannsvei 65**
**N-1364 Hvalstad (NO)**
Inventor : **Ramdahl, Thomas**
**Lijordveien 15**
**N-1343 Eiksmarka (NO)**
Inventor : **Moen, Vidar**
**Meierkollen 33**
**N-3727 Skien (NO)**

(74) Representative : **Allam, Peter Clerk**
**LLOYD WISE, TREGEAR & CO.**
**Norman House**
**105-109 Strand**
**London WC2R 0AE (GB)**

## (54) Aromatic imine compounds, pharmaceutical composition thereof, useful as protein synthesis inhibitors.

(57) New compounds useful as anti-cancer agents and as agents useful for the treatment of illnesses arising due to an abnormally elevated cell proliferation rate, having the general formula (I) :

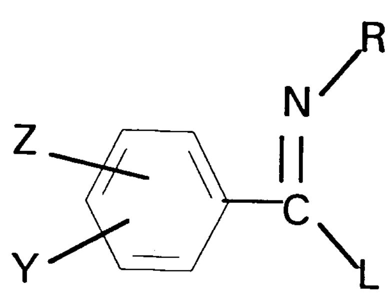

wherein :
L is hydrogen or deuterium ;
R may be alkyl, alkenyl, alkynyl, having from 1-20C atoms, $OR_1$, wherein $R_1$ is H or alkyl with 1-4 carbon atoms, $COR_2$ wherein $R_2$ is OH or $OR_5$ wherein $R_5$ is alkyl with 1-4 carbon atoms, NR'R" wherein R' and R" may be the same or different and may be H or alkyl with 1-4 carbon atoms ; $R_3XR_4$ wherein $R_3$ and $R_4$ may be the same or different and may be alkyl with 1-20 carbon atoms and X may be O, NH, S ; substituted or unsubstituted phenyl, unsubstituted or substituted heterocyclic ring, amino, monoalkyl amino or dialkyl amino wherein the alkyl groups have 1-20 C atoms, all said groups may be further substituted.

Z and Y, which may be the same or different, are H, D, alkyl with 1-4 carbon atoms, halogen, nitro, amino, monoalkyl amino or dialkyl amino wherein the alkyl groups have 1-4 C atoms, or OR wherein R may be H or alkyl with 1-4 carbon atoms, or $CF_3$, - $CR_1$=O, wherein $R_1$ may be H, D, alkyl with 1-4 carbon

EP 0 609 032 A1

atoms, or $OR_2$, wherein $R_2$ may be H or an alkyl with 1-4 carbon atoms, -CN, $R_3XR_4$, wherein $R_3$ and $R_4$ may be the same or different and may be alkyl with 1-20 carbon atoms and X may be O, NH or S ;
and pharmaceutically acceptable salts thereof.

The present invention concerns new chemical compounds which are useful as anticancer agents or as agents which may be used for combatting illnesses which arise due to an elevated cell proliferation.

Technical Background

The presently used anti-cancer agents are predominantly cytotoxic in action. Although these agents have given good results in the treatment of some cancers, they often produce severe and unacceptable side-effects limiting the possibility of an effective treatment. Furthermore, in several types of cancer, chemotherapy has so far proven of limited value since no established cytostatic drug improves the prognosis of the patient. There is thus a need for new anti-cancer agents having fewer side effects and having a more selective action on the cancer cells.

It is known among other from EP215395, J63264411, J88009490, J55069510 and EP283139 that benzaldehydes and derivatives thereof exhibit a selective anti-cancer effect. This effect is predominantly due to an inhibition of the protein synthesis of the cell.

In solid tumours a reduced protein synthesis may result in a lack of vital proteins which lead to cell death. In normal cells there is a potential capacity for protein synthesis which is higher than in most cancer cells of solid tumours. This is demonstrated by comparison of the cell cycle duration in normal stem cells, which is often below 10 hours, and that of most cancer cells of solid tumours, which is typically 30-150 hours (see Gavosto and Pileri in: The Cell Cycle and Cancer. Ed. :Baserga, Marcel Dekker Inc., N.Y. 1971, pp 99). Since cells, as an average, double their protein during the cell cycle, this means that protein accumulation is greater in growth-stimulated normal cells than in most types of cancer cells.

Another difference which is of similar importance between normal and cancer cells is the following: While normal cells respond to growth-regulatory stimuli, cancer cells have a reduced or no such response. Thus, while normal cells, under ordinary growth conditions, may have a reserve growth potential, cancer cells have little or no such reserve. If a mild protein synthesis inhibition is imposed continuously over a long period of time on normal cells as well as on cancer cells it is probable that the two different types of cells will respond differently: Normal tissue may take into use some of its reserve growth potential and thereby maintain normal cell production. Cancer tissue however, has little or no such reserve. At the same time the rate of protein accumulation in most cancer cells is rather low (i.e. protein synthesis is only a little greater than protein degradation). Therefore a certain degree of protein synthesis inhibition could be just enough to render the tumour tissue imbalanced with respect to protein accumulation, giving as a result a negative balance for certain proteins. During continuous treatment for several days this will result in cell inactivation and necrosis in the tumour tissue while normal tissue is unharmed.

It is known from UK Patent application 9026080.3 that benzaldehyde compounds, previously known as anti-cancer agents may be used for combatting diseases resulting from an abnormally elevated level of cell proliferation, such as psoriasis, inflammatory diseases, rheumatic diseases and allergic dermatologic reactions.

Dermatologic abnormalities such as psoriasis are often characterised by rapid turnover of the epidermis. While normal skin produces approximately 1250 new cells/day/$cm^2$ of skin consisting of about 27,000 cells, psoriatic skin produces 35,000 new cells/day/$cm^2$ from 52,000 cells. The cells involved in these diseases are however "normal" cells reproducing rapidly and repeatedly by cell division. As compared to psoriatic skin where most stem cells proliferate, very few cells in normal skin proliberate. However, those stem cells in normal skin that do not proliferate are in a resting stage from which they can be stimulated to proliferation. When the protein syntesis is reduced by specific inhibition in psoriatic as well as normal skin, the psoriatic skin will have little possibility of recruiting resting stem cells for proliferation as compared to normal skin, and thus a specific growth inhibition in the psoriatic skin may be obtained.

The inhibition of protein synthesis achieved with benzaldehydes and derivatives thereof also induces a prolonged cell cycle duration such that a reduction of the cell production as well as a reduction of protein synthesis is achieved during treatment. Therefore diseases for which the symptomatic cause is an enhanced cell proliferation rate can be treated with benzaldehydes or derivatives thereof without this leading to cell death - a condition unwanted since the cells involved are 1 ormal cells with an abnormal cell proliferation rate.

It has now surprisingly been found that imines of formula I will exhibit an even stronger protein synthesis inhibition, and, even more important, they exhibit an inhibitory effect on the protein synthesis in cell types on which the compounds of the above mentioned prior art do not have any effect.

Examples of diseases which may be treated with the compounds according to this invention are cancer, rheumatoid arthritis, psoriatic arthritis, systemic lupus erythematosus (SLE), discoid lupus erythematosus (DLE), acne, Bechterew's arthritis, systemic scleroderma and seborrhea.

**Detailed description**

The compounds of the present invention have the general formula (I):

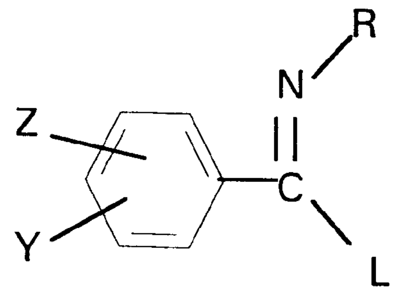

wherein:
L is hydrogen or deuterium;

R may be alkyl, alkenyl, alkynyl, having from 1-20 C atoms, $OR_1$, wherein $R_1$ is H or alkyl with 1-4 carbon atoms, $COR_2$ wherein $R_2$ is OH or $OR_5$ wherein $R_5$ is alkyl with 1-4 carbon atoms, NR'R" wherein R' and R" may be the same or different and may be H or alkyl with 1-4 carbon atoms; $R_3XR_4$ wherein $R_3$ and $R_4$ may be the same or different and may be alkyl with 1-20 carbon atoms and X may be O, NH, S; substituted or unsubstituted phenyl, unsubstituted or substituted heterocyclic ring, amino, monoalkyl amino or dialkyl amino wherein the alkyl groups have 1-20 C atoms, all said groups may be further substituted.

Z and Y, which may be the same or different, are H, D, alkyl with 1-4 carbon atoms, halogen, nitro, amino, monoalkyl amino or dialkyl amino wherein the alkyl groups have 1-4 C atoms, or OR wherein R may be H or alkyl with 1-4 carbon atoms, or $CF_3$, $-CR_1 =O$, wherein $R_1$ may be H, D, alkyl with 1-4 carbon atoms, or $OR_2$, wherein $R_2$ may be H or an alkyl with 1-4 carbon atoms, -CN, $R_3XR_4$, wherein $R_3$ and $R_4$ may be the same or different and may be alkyl with 1-20 carbon atoms and X may be O, NH or S;
and pharmaceutically acceptable salts thereof.

The phenyl ring of the compounds of formula I may carry one or several groups Z, and Y, at the most five Z + Y groups.

When R being any of the above groups is further substituted, the substituents will preferably be chosen from the group comprising alkyl of 1-4 carbon atoms, halogen, nitro, amino, OR' wherein R' is H or alkyl of 1-4 carbon atoms, or $CF_3$.
When R is alkyl, alkynyl or alkenyl having from 1-20 carbon atoms, a particular optional substituent is alkyl of 1-4 carbon atoms.

When any group is alkyl with 1-4 carbon atoms it is most preferred methyl, ethyl or isopropyl. Preferred alkyl groups of 1-20 carbon atoms will be the groups having 16-20 carbon atoms, especially 18-20 carbon atom which preferred may be mono unsaturated.

The alkylgroups may be branched or unbranched, cyclic or acyclic, carrying one or several double bonds. The halogens may be any of chlorine, bromine, iodine or fluorine.

**Preparation**

The imine compounds of formula (I) according to this invention may be prepared by well-known procedures by reacting the corresponding aldehyde with a primary amine, $NH_2R$, said group R may be alkyl, alkenyl, alkynyl, having from 1-20 carbon atoms, amino, monoalkyl amino or dialkyl amino wherein the alkyl groups have 1-20 carbon atoms, said groups may be further substituted.

There are two preferred methods for preparing the imines according to the present invention and these are described below using the most preferred solvents, reaction media, apparatuses and the like. A person skilled in the art will see (bvious ways of varying the described methods for instance by use of other solvents or the like.

<u>Method I</u>

Equivalent amounts of aldehyde and amine are dissolved in toluene in an apparatus comprising a Dean-Stark water separator. The composition is refluxed until stochiometric amounts of water have been collected or overnight. The reaction mixture is evaporated on the rotavapor and the residue is taken up in dichloromethane and washed with 5 % sodium bicarbonate. The organic phase is dried (Mg $SO_4$) filtered and evaporated. The raw product can be worked up by different routes, depending on different factors, such as stability and volatility. If possible, a vacuum distillation can be performed. Less volatile compounds are recrystallized (hexane/ethyl acetate) or purified by column chromatography (Silica, triethyl amine/chloroform or pyridine)

Method II

If the amine is insoluble in toluene, the aldehyde and amine in equivalent amounts or with a surplus of aldehyde, are mixed with water/methanol 1:3 . The mixture is boiled under reflux until an acceptable conversion has taken place. Unreacted amine is filtered off and the filtrate is evaporated on the rotavapor. The residue is worked up as described under method I.

Generally the temperature and solvents used will depend on the reactivity and solubility of the reactants.

The compounds of formula I wherein L is deuterium may be prepared as described above, but starting with deuterated benzaldehyde or benzaldehyde derivatives, which may carry one or more further substituents on the phenyl ring.

The following examples are illustrative of how the compounds of the present invention may be prepared.

**EXAMPLE 1**

**PREPARATION OF 11-(BENZYLIDENE-$d_1$-AMINO) - UNDECANOIC ACID**

Benzaldehyde-$d_1$(10.0 g, 0.093 mol) and 11-aminoundecanoic acid (18.8 g, 0.0933 mol) were mixed with toluene (300 ml) in a 500 ml three-necked flask equipped with a Dean Stark water trap.

The reaction mixture was refluxed with stirring overnight and the solvent removed by evaporation, leaving a slightly yellow solid, 28.6 g. 5.0 g crude product was recrystallized from hexane (150 ml) giving 2.84 g finely divided white powder, m.p. 60-61.5°C.

The identity was confirmed by NMR-spectroscopy.

**EXAMPLE 2**

**PREPARATION OF 6(BENZYLIDENE-$d_1$-AMINO) - HEXANOIC ACID**

Benzaldehyde-$d_1$(10.0 g , 0.093 mol) and 6-aminohexanoic acid (12.2 g, 0.0933 mol) were mixed with toluene (300 ml) in a 500 ml three-necked flask equipped with a Dean Stark water trap.

The reaction mixture was refluxed with stirring overnight and the solvent removed by evaporation, leaving a slightly yellow oil which solidified upon standing, 22.3 g. 5.0 g crude product was recrystallized from ethylacetate (30 ml) and hexane (120 ml) giving 2.9 g colorless prism-shaped crystals, m.p. 67-71°C.

The identity was confirmed by NMR-spectroscopy.

**EXAMPLE 3**

**PREPARATION OF 6-(3-NITROBENZYLIDENE-AMINO) - HEXANOIC ACID**

3-Nitrobenzaldehyde (10.0 g, 0.066 mol) and 6-aminohexanoic acid (8.2 g, 0.066 mol) were mixed with toluene (300 ml) in a 500 ml three-necked flask equipped with a Dean Stark water trap.

The reaction mixture was refluxed with stirring overnight and the solvent removed by evaporation, leaving a yellow solid, 16.4 g. 5.0 g crude product was recrystallized from a mixture of ethylacetate (30 ml) and hexane (120 ml) giving 3.12 g finely divided powder, tinged with yellow, m.p. 84.5-86.5°C.

The identity was confirmed by NMR-spectroscopy.

**EXAMPLE 4**

**PREPARATION OF 11-(4-CARBOMETHOXYBENZYLIDENE-AMINO) - UNDECANOIC ACID**

Methyl-4-formylbenzoate (10.0 g, 0.061 mol) and 11-aminoundecanoic acid (12.3 g, 0.061 mol) were mixed with toluene (300 ml) in a 500 ml three-necked flask equipped with a Dean Stark water trap.

The reaction mixture was refluxed with stirring overnight and the solvent removed by evaporation, leaving a white solid, 21.0 g. 5.0 g crude product was recrystallized from hexane (150 ml) giving 2.12 g finely divided

white powder, m.p. 77-78.5°C.

The identity was confirmed by NMR-spectroscopy.

**EXAMPLE 5**

**PREPARATION OF 5-(BENZYLIDENE-$d_1$-AMINO)-3-OXA-PENTAN-1-OL**

Benzaldehyde-$d_1$(10.0 g, 0.093 mol) and 2-(2-aminoethoxy)-ethanol (9.8 g, 0.093 mol) were dissolved in toluene (300 ml) in a 500 ml three-necked flask equipped with a Dean Stark water trap.

The reaction mixture was refluxed with stirring overnight and the solvent removed by evaporation. The residue was taken up in chloroform (100 ml) and washed with 5% $NaHCO_3$ solution (3 x 25 ml). The organic phase was dried ($MgSO_4$), filtered and evaporated. The crude product was destilled under vacuum giving a colorless oil, b.p. 102-104°C/0.15 mbar. The yield was 13.4 g, 74% of the theoretical.

The identity was confirmed by NMR- spectroscopy.

**EXAMPLE 6**

**PREPARATION OF 4-(BENZYLIDENE-$d_1$-AMINO) - BUTAN-1-OL**

Benzaldehyde-$d_1$ (10.0 g, 0.093 mol) and 4-amino-1-butanol (8.3 g, 0.093 mol) were dissolved in toluene (300 ml) in a 500 ml three-necked flask equipped with a Dean Stark water trap.

The reaction mixture was refluxed with stirring overnight and the solvent removed by evaporation. The residue was taken up in chloroform (100 ml) and washed with 5% $NaHCO_3$ solution (3 x 25 ml). The organic phase was dried ($MgSO_4$), filtered and evaporated. The crude product was distilled in vacuum giving a colorless oil, b.p. 124-126°C/1.0 mbar. The yield was 6.0 g, 36% of the theoretical.

The identity was confirmed by NMR-spectroscopy.

**EXAMPLE 7**

**PREPARATION OF 4-(3-NITROBENZYLIDENE-AMINO)-BUTAN-1-OL**

3-Nitrobenzaldehyde (10.0 g, 0.066 mol) and 4-amino-1-butanol (5.9 g, 0.066 mol) were dissolved in toluene (300 ml) in a 500 ml three-necked flask equipped with a Dean Stark water trap.

The reaction mixture was refluxed with stirring overnight and the solvent removed by evaporation. The residue was taken up in chloroform (100 ml) and washed with 5% $NaHCO_3$ solution (3 x 25 ml). The organic phase was dried ($MgSO_4$), filtered and evaporated. The crude product was a yellow oil which solidified upon standing. This solid was recrystallized from ethylacetate/hexane 1:4 giving needle-shaped crystals with a yellow tinge, m.p. 55-58°C. The yield was 12.1 g, 82% of the theoretical.

The identity was confirmed by NMR- spectroscopy.

**EXAMPLE 8**

**PREPARATION OF5-(4-CARBOMETHOXYBENZYLIDENE-AMINO) -3-OXAPENTAN-1-OL**

Methyl-4-formylbenzoate (10.0 g, 0.061 mol) and 2-(2-aminoethoxy)-ethanol (6.4 g, 0.061 mol) were dissolved in toluene (300 ml) in a 500 ml three-necked flask equipped with a Dean Stark water trap.

The reaction mixture was refluxed with stirring overnight and the solvent removed by evaporation. The residue was taken up in chloroform (100 ml) and washed with 5% $NaHCO_3$ solution (3 x 25 ml). The organic phase was dried ($MgSO_4$), filtered and evaporated. The crude product was an orange oil of acceptable purity, about 98%, as shown by GC-analysis.

The identity was confirmed by NMR-spectroscopy.

**EXAMPLE 9**

**PREPARATION OF 2-(BENZYLIDENE-$d_1$-AMINO)-ETHANOL**

Benzaldehyde-$d_1$ (10.7 g, 0.10 mol) and ethanolamine (6.1 g, 0.10 mol) were dissolved in toluene (300 ml) in a 500 ml three-necked flask equipped with a Dean Stark water trap. The reaction mixture was refluxed under

stirring for 24 hours, and the solvent removed by evaporation.

The residue was taken up in dichloromethane (100 ml) and washed with 5% $NaHCO_3$ solution. The crude product was distilled under vacuum (< 1 mbar) to give a colourless oil with narrow boiling range, which solidified upon standing in the refrigerator.

The product was identified as the imine tautomer neat (IR) as well as in acetone-$d_6$ solution (NMR).

The undeuterated analog was prepared in a separate experiment, and the imine-proton shown to resonate at 8.30 ppm. This signal was absent in the spectrum of the deuterated compound, proving the d-grade to be excellent.

## EXAMPLE 10

### PREPARATION OF 1-(3-NITROBENZYLIDENE-$d_1$-AMINO)-3- (DIMETHYLAMINO)-PROPANE

3-Nitrobenzaldehyde-$d_1$(5.0 g, 0.033 mol) and N, N-dimethyl trimethylenediamine (3.34 g, 0.033 mol) were dissolved in toluene (200 ml) in a three-necked flask equipped with a Dean Stark water trap.

The reaction mixture was refluxed with stirring overnight and the solvent removed by evaporation. The residue was taken up in dichloromethane (100 ml) and washed with 5% $NaHCO_3$ solution. The organic phase was dried over $MgSO_4$, filtered and evaporated. The crude product was treated on a Lobar LC silica column, eluting with 5% triethylamine in chloroform. Collecting fractions from four separate runs gave 2.8 g product, 36% of the theoretical yield.

The identity was confirmed by NMR spectroscopy.

## EXAMPLE 11

### PREPARATION OF 5-(BENZYLIDENE-$d_1$-AMINO)-URACIL

Benzaldehyde-$d_1$ (10.0 g, 0.093 mol) and 5-aminouracil (6.0 g, 0.047 mol) were mixed with methanol (350 ml) and water (150 ml) in a three-necked flask.

The reaction mixture was refluxed for several days, filtered whilst warm and the filtrate concentrated by evaporation. 0.5 g samples of the crude product was boiled in pyridine (10 ml), filtered and the filtrate treated on a Lobar LC silica column, eluting with pyridine. The yellow fractions were collected and evaporated. The last traces of pyridine were removed by an oil pump, leaving a white solid.

The identity was confirmed by GC/MS and NMR-spectroscopy.

## EXAMPLE 12

### PREPARATION OF 2-(BENZYLIDENE-$d_1$-AMINO)-2-METHYLPROPANOL

Benzaldehyde-$d_1$ (10.0 g, 0.093 mol) and 2-amino-2-methyl-1-propanol (8.31 g, 0.093 mol) were dissolved in toluene (300 ml) in a three-necked flask equipped with a Dean Stark water trap.

The reaction mixture was refluxed with stirring overnight and the solvent removed by evaporation. The residue was taken up in dichloromethane (100 ml) and washed with 5% $NaHCO_3$ solution. The organic phase was dried over $MgSO_4$, filtered and evaporated. The crude product was distilled under vacuum (84-86°C/1.8 mbar) to give a white solid, m.p. 68-74°C. Yield: 11.8 g, 71% of the theoretical.

$^1H$ and $^{13}C$ NMR showed that the dissolved product exists as a tautomeric equilibrium between the imine and the corresponding oxazolidine, the position of which depends on the solvent. IR (KBr tablet) showed a predominant C=N stretch frequency as expected for an imine.

## EXAMPLE 13

### PREPARATION OF 1-(BENZYLIDENE-$d_1$-AMINO)-2-(2-DEUTERO-2- PHENYLOXAZOLIDINE-3-YL)-ETHANE

Benzaldehyde-$d_1$ (20.0 g, 0.187 mol) and 2-(2-aminoethylamino)-ethanol (9.7 g, 0.093 mol) were dissolved in toluene (300 ml) in a 500 ml three-necked flask equipped with a Dean Stark water trap.

The reaction mixture was refluxed with stirring overnight, and the solvent removed by evaporation. The residue was taken up in dichloromethane (100 ml) and washed with 5% $NaHCO_3$ solution. The organic phase was dried over $MgSO_4$, filtered and evaporated. The crude product was distilled under vacuum to give a yellow

oil, b.p. 150-152°C/0.01 mbar. Yield: 14.9 g, 57% of the theoretical.

The identity was confirmed by NMR and GC/MS spectroscopy.

## EXAMPLE 14

### PREPARATION OF 1-(BENZYLIDENE-$d_1$-AMINO)-3-(DIMETHYLAMINO)- PROPANE

Benzaldehyde-$d_1$ (10.0 g, 0.093 mol) and 3-dimethyl-aminopropylamine (9.5 g, 0.093 mol) were dissolved in toluene (300 ml) in a 500 ml three-necked flask equipped with a Dean Stark water trap.

The reaction mixture was refluxed with stirring overnight and the solvent removed by evaporation. The residue was taken up in dichloromethane (100 ml) and washed with 5% $NaHCO_3$ solution. The crude product was distilled under vacuum (< 1 mbar) to give a colourless oil of narrow boiling range (Yield: 11.4 g, 64% of theoretical).

The identity was confirmed by GC/MS and NMR spectroscopy. NMR indicated the degree of deuteration to be excellent.

The structural formulas of the different compounds are presented in Table 1.

### Biological experiments

In the following in vitro experiments, the rate of protein synthesis was measured for a compound from the prior art, which is deuterated sodium 5,6-O-benzylidene-L-ascorbate (sodium salt of zilascorb($^2$H)), compound 15, and for imine compounds according to the present invention. Also the effects of two preferred compounds of the invention, compounds 3 and 7 are shown in cell systems wherein the prior art compound does not have any effect.

### Cell Culturing Techniques and Synchronization

Human cells of the established line NHIK 3025, originating from a cervical carcinoma in situ (Nordbye, K. and Oftebro, R., Exp. Cell Res., 58: 458, (1969)), (Oftebro, R. and Nordbye, K., Exp. Cell Res., 58: 459-460, (1969)) were cultivated in medium E2a (Puck et al., J. Exp. Med., 106: 145-165, (1957)) supplemented with 20% human (prepared at the laboratory) and 10% horse serum (Grand Island Biological Co.).

Balb 3T3-A cells were isolated by Aaronsen and Todaro (J. Cell Physiol. 72, (1968)) 41-148) and were provided by Dr. T.Ege (Karolinska Institutet, Stockholm) in 1980 and have since been cultivated in MEM medium (Hank's salts) supplemented with 15% newborn bovine serum.

V79 379-A cells were provided by Dr. Laszlo Révész (Karolinska Institutet, Stockholm) in 1976 and have since been cultivated in MEM medium (Hank's salts) supplemented with 15% newborn bovine serum. These cells are a clone of the line V79, isolated from Chinese hamster normal lung tissue and established in culture by Ford and Yerganian (see J. Natl. Cancer Inst. 21, (1958) 393-425).

PANC-1 cells were purchased from the American Type Culture Collection (ATCC) in 1990. These cells were derived from a human adenocarcinoma of ductal origin (Lieber et al, Int. J. Cancer 15, (1975) 741-747).

The cells are routinely grown as monolayers in tissue culture flasks. The cells were kept in continuous exponential growth by frequent reculturing, i.e. every second or third day.

During reculturing as well as during experiments the cells were kept in a walk-in incubator at 37°C.

### Protein Synthesis:

The rate of protein synthesis was calculated as described previously (Rønning et al., J. Cell Physiol., 107: 47-57, (1981)). Briefly, cellular protein was labeled to saturation during a 2-day preincubation with [$^{14}$C]-valine of constant specific radioactivity (0.5 Ci/mol) prior to the experiment. This was achieved by using a high concentration of valine so that the dilution of [$^{14}$C]- valine by intracellular valine and by proteolytically generated valine will be negligible (Rønning et al., Exp. Cell Res., 123: 63-72, (1979)), thus keeping the specific radioactivity at a constant level. The rate of protein synthesis was calculated from the incorporation of [$^3$H]-valine of constant specific activity. The incorporated measurements were related to the total of [$^{14}$C]-radioactivity in protein at the beginning of the respective measurement periods and expressed as the percentage per hr (Rønning et al., J. Cell. Physiol., 107: 47-57, (1981)).

**Description of Figures**

In Figure 1 the rate of protein synthesis (as % of control rate) is shown in relation to the concentration of imine used in the treatment of human NHIK 3025/MEM cervix carcinoma cells cultivated in vitro. The treatment period was for 1 hour during which the cell culture medium contained [$^3$H]-valine in addition to the test compound. As compared to the effect of the prior art compound 15, the protein synthesis was inhibited to a greater degree by compounds 1, 2, 3, and 4, with compounds 3 and 4 inducing the strongest inhibition on protein synthesis.

In Figure 2 the rate of protein synthesis (as % of control rate) is shown in relation to the concentration of imine used in the treatment of human NHIK 3025/MEM cervix carcinoma cells cultivated in vitro. The treatment period was for 1 hour during which the cell culture medium contained [$^3$H]-valine in addition to the test compound. As compared to the effect of the prior art compound 15, the protein synthesis was inhibited to a greater degree by compounds 5, 6 and 7, with compound 7 inducing the strongest inhibition on protein synthesis.

In Figure 3, the effect of two imine compounds, 3 and 7, according to the invention and the effect of the prior art compound 15 on the protein synthesis of murine B16 melanoma cells is shown. As appears the two compounds of the invention have a much stronger protein synthesis inhibiting effect.

In Figure 4 the effect of two preferred imines according to the invention, compounds 3 and 7 , on the rate of protein synthesis in the non-malignant cell line V79 (Chinese hamster lung cells). Compound 15 induced little or no inhibition of the protein synthesis in these cells. Compounds 3 and 7, however, did significantly inhibit protein synthesis in V79 cells. Furthermore, compound 7 is slightly more effective than compound 3.

In Figure 5 the effect of compounds 3 and 7 on the rate of the protein synthesis of 3T3 cells (derived from mouse embryo) is shown. Compounds 3 and 7 induce strong protein synthesis inhibition, while the prior art compound 15 had little effect.

In Figures 6a) and b) the human pancreatic carcinoma cell line PANC-1, cultivated in the medium MEM (with new born calf serum), was used . While compound 15 induced little protein synthesis inhibiting activity (Figure 6a), compound 14 did show a clear protein synthesis inhibiting effect (Figure 6b). The data show that compound 14 also induces a cell inactivating effect at concentrations above 2 mM as shown in the decline in [$^{14}$C]-radioactivity (a decrease in [$^{14}$C]-radioactivity is a sign of cell death). However, for concentrations of 2mM and less (non-toxic doses) compound 14 induces strong protein synthesis inhibition.

In Figures 7a) and b) the human pancreatic carcinoma cell line PANC-1, cultivated in the medium E2a (with human and horse serum), was used . While compound 15 induced little protein synthesis inhibiting activity (Figure 7a), compound 14 did show a clear protein synthesis inhibiting effect (Figure 7b). The data show that compound 14 also induces a cell inactivating effect at concentrations above 2 mM as shown in the decline in [$^{14}$C]-radioactivity (a decrease in [$^{14}$C]-radioactivity is a sign of cell death). However, for concentrations of 2mM and less (non-toxic doses) compound 14 induces strong protein synthesis inhibition.

The Figures 6a) and b) and 7a) and b) clearly show that the compound 14 is a far more efficient protein synthesis inhibitor than the prior art compound 15 irrespective of the medium used.

Several other experiments have shown the same type of effect.

According to present invention the compounds of formula I may be administered to a patient in need of anti-cancer treatment or to a patient suffering from diseases which arise due to an abnormally elevated cell proliferation.

For this purpose the compounds may be formulated in any suitable manner for administration to a patient either alone or in admixture with suitable pharmaceutical carriers or adjuvants. It is especially preferred to prepare the formulations for systemic therapy either as oral preparations or parenteral formulations. Suitable enteral preparations will be tablets, capsules, e.g. soft or hard gelatine capsules, granules, grains or powders, syrups, suspensions, solutions or suppositories. Such will be prepared as known in the art by mixing one or more of the compounds of formula I with non-toxic, inert, solid or liquid carriers.

Suitable parental preparations of the compounds of formula I are injection or infusion solution.

When administered topically the compounds of formula I may be formulated as a lotion, salve, cream, gel, tincture, spray or the like containing the compounds of formula I in admixture with non-toxic, inert, solid or liquid carriers which are usual in topical preparations. It is especially suitable to use a formulation which protects the active ingredient against air, water and the like.

The preparations can contain inert or pharmacodynamically active additives. Tablets or granulates e.g. can contain a series of binding agents, filler materials, carrier substances and/or diluents. Liquid preparations may be present, for example, in the form of a sterile solution. Capsules can contain a filler material or thickening agent in addition to the active ingredient. Furthermore, flavour-improving additives as well as the substances usually used as preserving, stabilizing, moisture-retaining and emulsifying agents, salts for varying the osmotic pressure, buffers and other additives may also be present.

The dosages in which the preparations are administrered can vary according to the indication, the mode of use and the route of administration, as well as to the requirements of the patient. In general a daily dosage for a systemic therapy for an adult average patient in need of anti-cancer treatment will be about 0.01-500mg/kg body weight/day, preferably 0. 1-100mg/kg body weight/day.

The daily dosage for a systemic therapy for an adult average patient in need of treatment for elevated cell-proliferation will be about 0.1-50 mg/kg/day preferably 1-15 mg/kg/day. For topic administration, the suitable salve or ointment can contain from 0.1-50% by weight of the pharmacetical formulation, especially 1-20%.

If desired the pharmaceutical preparation of the compound of formula I can contain an antioxidant, e.g. tocopherol, N-methyl-tocopheramine, butylated hydroxyanisole, ascorbic acid or butylated hydroxytoluene.

**TABLE 1**

| Compound No. | Structure | Name |
|---|---|---|
| 1 | 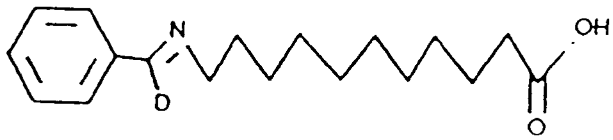 | 11-(Benzylidene-$d_1$-Amino) -Undecanoic Acid |
| 2 | 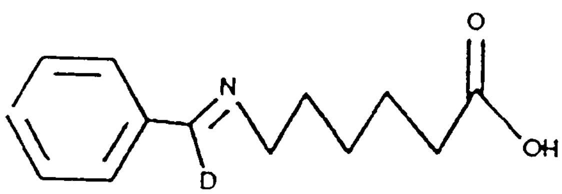 | 6-(Benzylidene-$d_1$-Amino) -Hexanoic Acid |
| 3 | 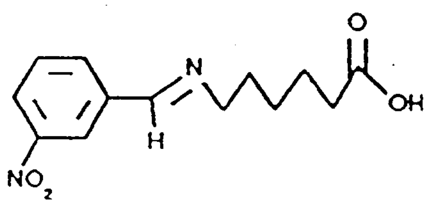 | 6-(3-Nitrobenzylidene-Amino)-Hexanoic Acid |
| 4 | 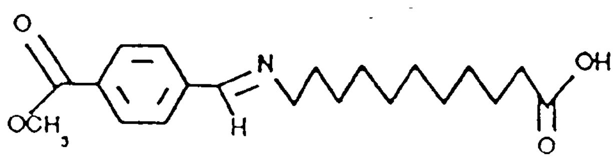 | 11-(4-Carbomethoxy-benzylidene-Amino) -Undecanoic Acid |
| 5 | 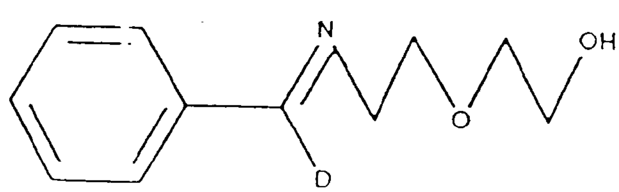 | 5-(Benzylidene-$d_1$-Amino)-3-Oxa-Pentan-1-ol |

**TABLE 1 continued**

| Compound No. | Structure | Name |
|---|---|---|
| 6 | 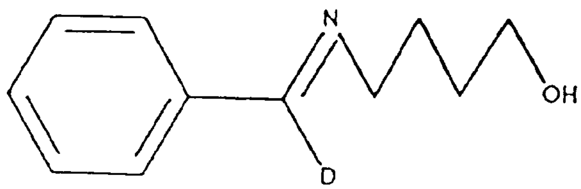 | 4-(Benzylidene-$d_1$-Amino) -Butan-1-ol |
| 7 | 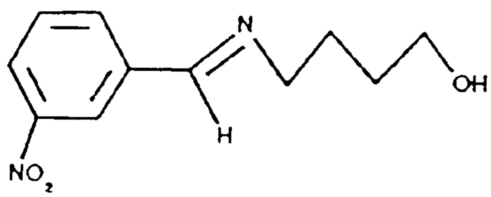 | 4-(3-Nitrobenzylidene-Amino)-Butan-1-ol |
| 8 | 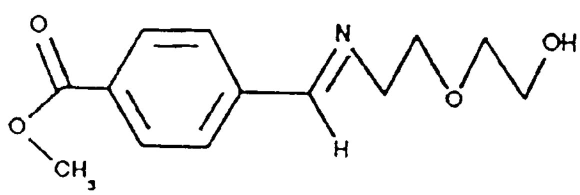 | 5-(4-Carbomethoxy-benzylidene-Amino)-3-Oxa-Pentan-1-ol |
| 9 | 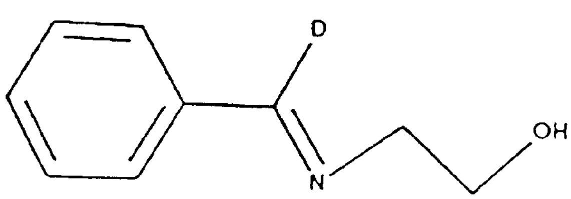 | 2-(Benzylidene-$d_1$-Amino)-Ethanol |
| 10 | 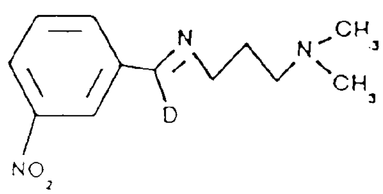 | 1-(3-Nitrobenzylidene-$d_1$-Amino)-3-(Dimethylamino)-Propane |

**TABLE 1 continued**

| Compound No. | Structure | Name |
|---|---|---|
| 11 | 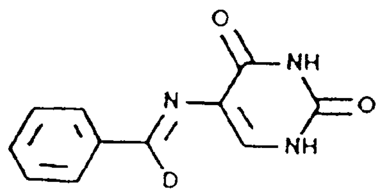 | 5-(Benzylidene-$d_1$-Amino)-Uracil |
| 12 | 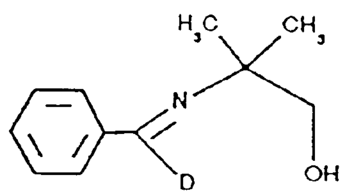 | 2-(Benzylidene-$d_1$-Amino)-2-Methyl-propanol |
| 13 | 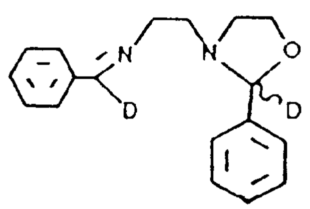 | 1-(Benzylidene-$d_1$-Amino)-2-(2-Deutero-2-Phenyloxazolidine-3-yl)-Ethane |
| 14 | 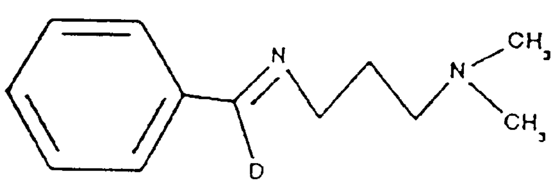 | 1-(Benzylidene-$d_1$-Amino)-3-(Dimethyl-amino)-Propane |
| 15 | 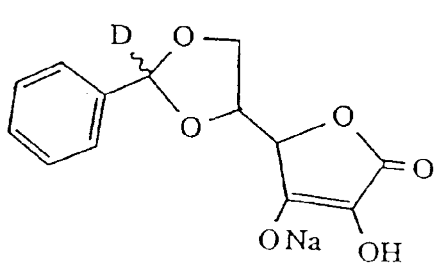 | Sodium 5,6-0-Benzylidene-$d_1$-L-Ascorbate |

## Claims

1. A compound of the general formula (I):

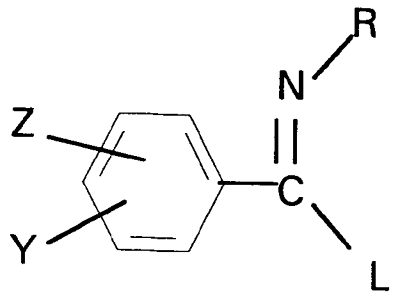

wherein:
L is hydrogen or deuterium;
R may be alkyl, alkenyl, alkynyl, having from 1-20 C atoms, $OR_1$, wherein $R_1$ is H or alkyl with 1-4 carbon atoms, $COR_2$ wherein $R_2$ is OH or $OR_5$ wherein $R_5$ is alkyl with 1-4 carbon atoms, NR'R" wherein R' and R" may be the same or different and may be H or alkyl with 1-4 carbon atoms; $R_3XR_4$ wherein $R_3$ and $R_4$ may be the same or different and may be alkyl with 1-20 carbon atoms and X may be O, NH, S; substituted or unsubstituted phenyl, unsubstituted or substituted heterocyclic ring, amino, monoalkyl amino or dialkyl amino wherein the alkyl groups have 1-20 C atoms, all said groups may be further substituted.

Z and Y, which may be the same or different, are H, D, alkyl with 1-4 carbon atoms, halogen, nitro, amino, monoalkyl amino or dialkyl amino wherein the alkyl groups have 1-4 C atoms, or OR wherein R may be H or alkyl with 1-4 carbon atoms, or $CF_3$, $-CR_1=O$, wherein $R_1$ may be H, D, alkyl with 1-4 carbon atoms, or $OR_2$, wherein $R_2$ may be H or an alkyl with 1-4 carbon atoms, -CN, $R_3XR_4$, wherein $R_3$ and $R_4$ may be the same or different and may be alkyl with 1-20 carbon atoms and X may be O, NH or S;
and pharmaceutically acceptable salts thereof.

2. A compound according to Claim 1, wherein L is D.

3. A compound according to Claim 1, wherein L is H.

4. A compound according to Claim 1, which is:
   11-(benzylidene-$d_1$-amino)-undecanoic acid;
   6-(benzylidene-$d_1$-amino)-hexanoic acid;
   6-(3-nitrobenzylidene-amino)-hexanoic acid;
   11-(4-carbomethoxy-benzylidene-amino)undecanoic acid;
   5-(benzylidene-$d_1$-amino)-3-oxa-pentan-1-ol;
   4-(benzylidene-$d_1$-amino)-butan-1-ol;
   4-(3-nitrobenzylidene-amino)-butan-1-ol;
   5-(4-carbomethoxy-benzylidene-amino)-3-oxa-pentan-1-ol;
   2-(benzylidene-$d_1$-amino)-ethanol;
   1-(3-nitrobenzylidene-$d_1$-amino)-3-(dimethylamino)-propane;
   5-(benzylidene-$d_1$-amino)-uracil;
   2-(benzylidene-$d_1$-amino)-2-methyl-propanol;
   1-(benzylidene-$d_1$-amino)-2-(2-deutero-2-phenyl-oxazolidine-3-yl)-ethane;
   1-(benzylidene-$d_1$-amino)-3-(dimethyl-amino)-propane; or
   sodium 5,6-0-benzylidene-$d_1$-L-ascorbate.

5. A pharmaceutical composition comprising a compound according to any preceding claim and a pharmaceutically acceptable carrier or adjuvant.

6. An orally administrable composition according to Claim 5.

7. A parenterally administrable composition according to Claim 5.

**8.** A compound according to any one of Claims 1-4 for use in the treatment of cancer or of diseases arising from an abnormally elevated cell-proliferation.

**9.** Use of a compound according to any one of Claims 1-4 for the manufacture of a therapeutic agent for the treatment of cancer or of diseases arising from an abnormally elevated cell-proliferation.

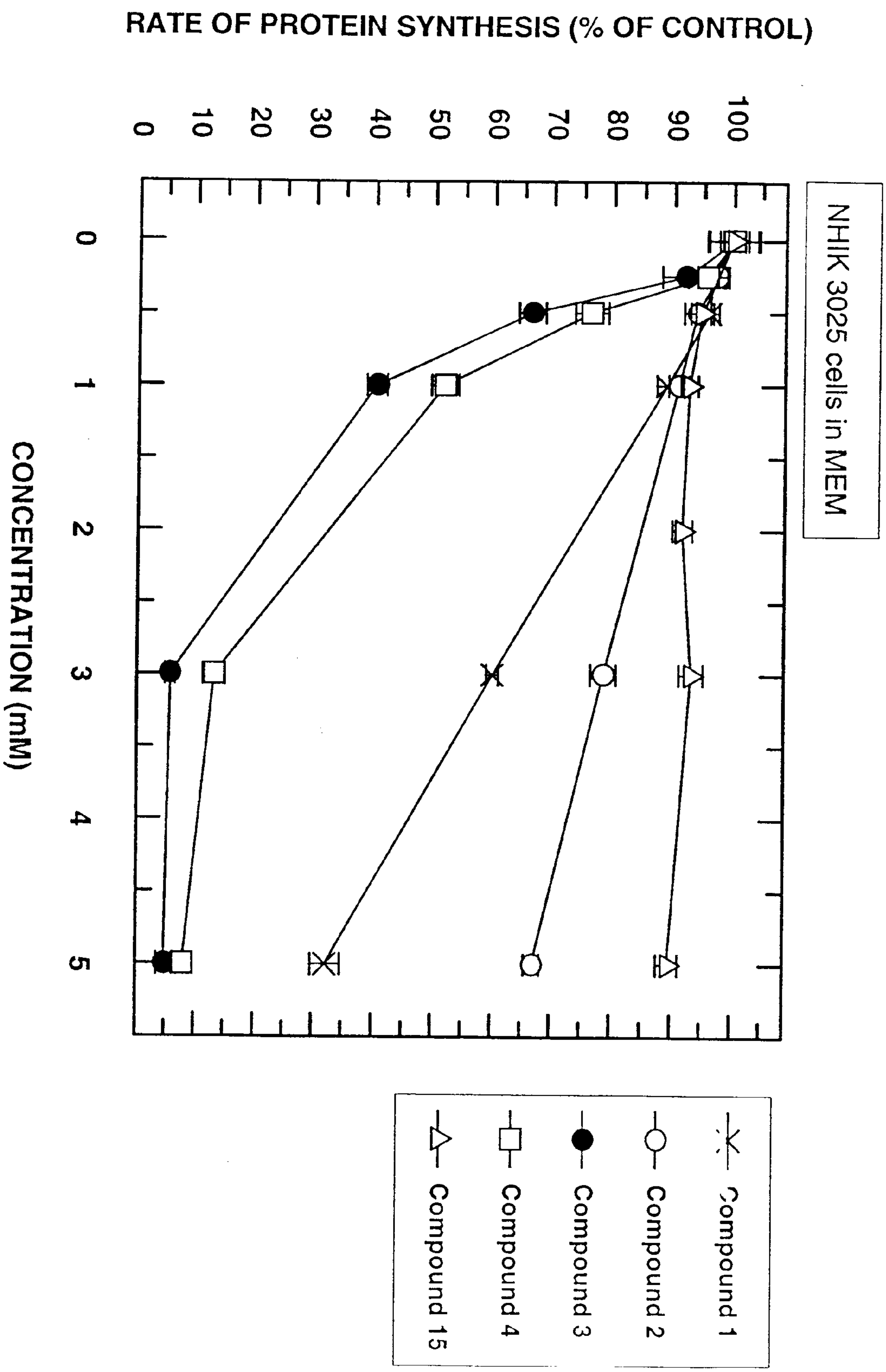
RATE OF PROTEIN SYNTHESIS (% OF CONTROL)
0
10
20
30
40
50
60
70
80
90
100
NHIK 3025 cells in MEM
0
1
2
3
4
5
CONCENTRATION (mM)
Compound 1
Compound 2
Compound 3
Compound 4
Compound 15

FIGURE 1

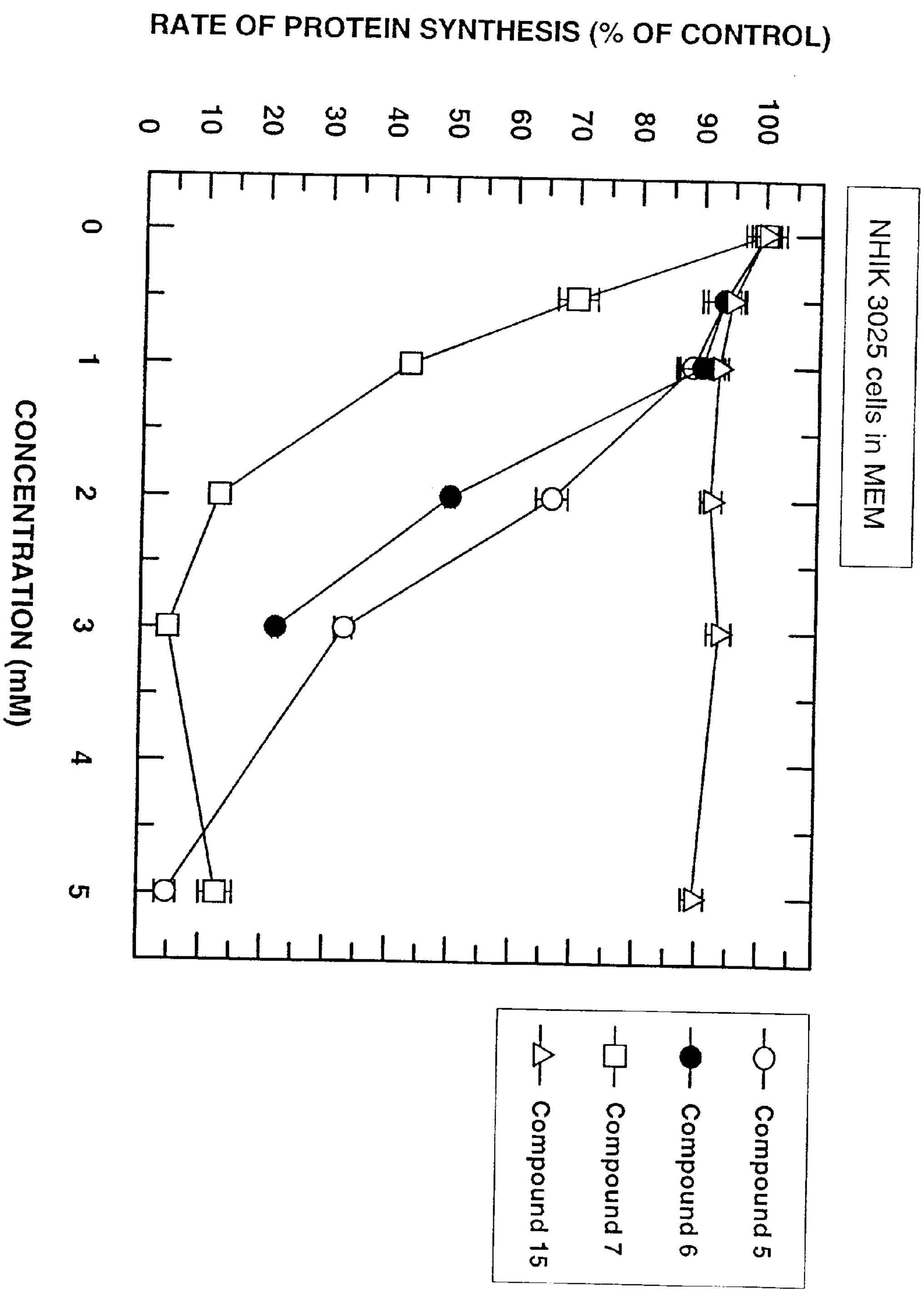
RATE OF PROTEIN SYNTHESIS (% OF CONTROL)
0
10
20
30
40
50
60
70
80
90
100
NHIK 3025 cells in MEM
0
1
2
3
4
5
CONCENTRATION (mM)
Compound 5
Compound 6
Compound 7
Compound 15

FIGURE 2

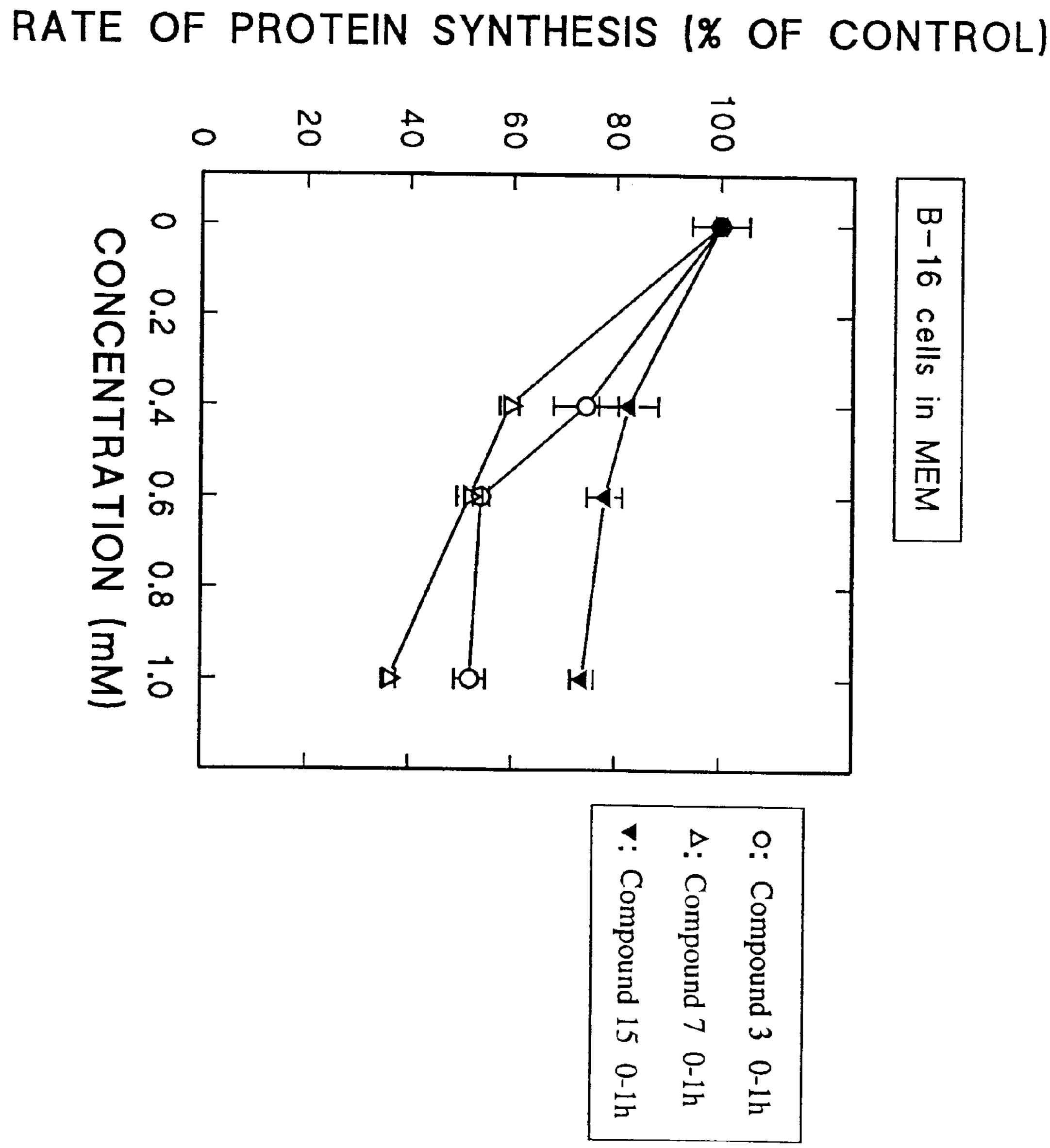
RATE OF PROTEIN SYNTHESIS (% OF CONTROL)
0
20
40
60
80
100
B-16 cells in MEM
0
0.2
0.4
0.6
0.8
1.0
CONCENTRATION (mM)
o: Compound 3 0-1h
Δ: Compound 7 0-1h
▼: Compound 15 0-1h

FIGURE 3

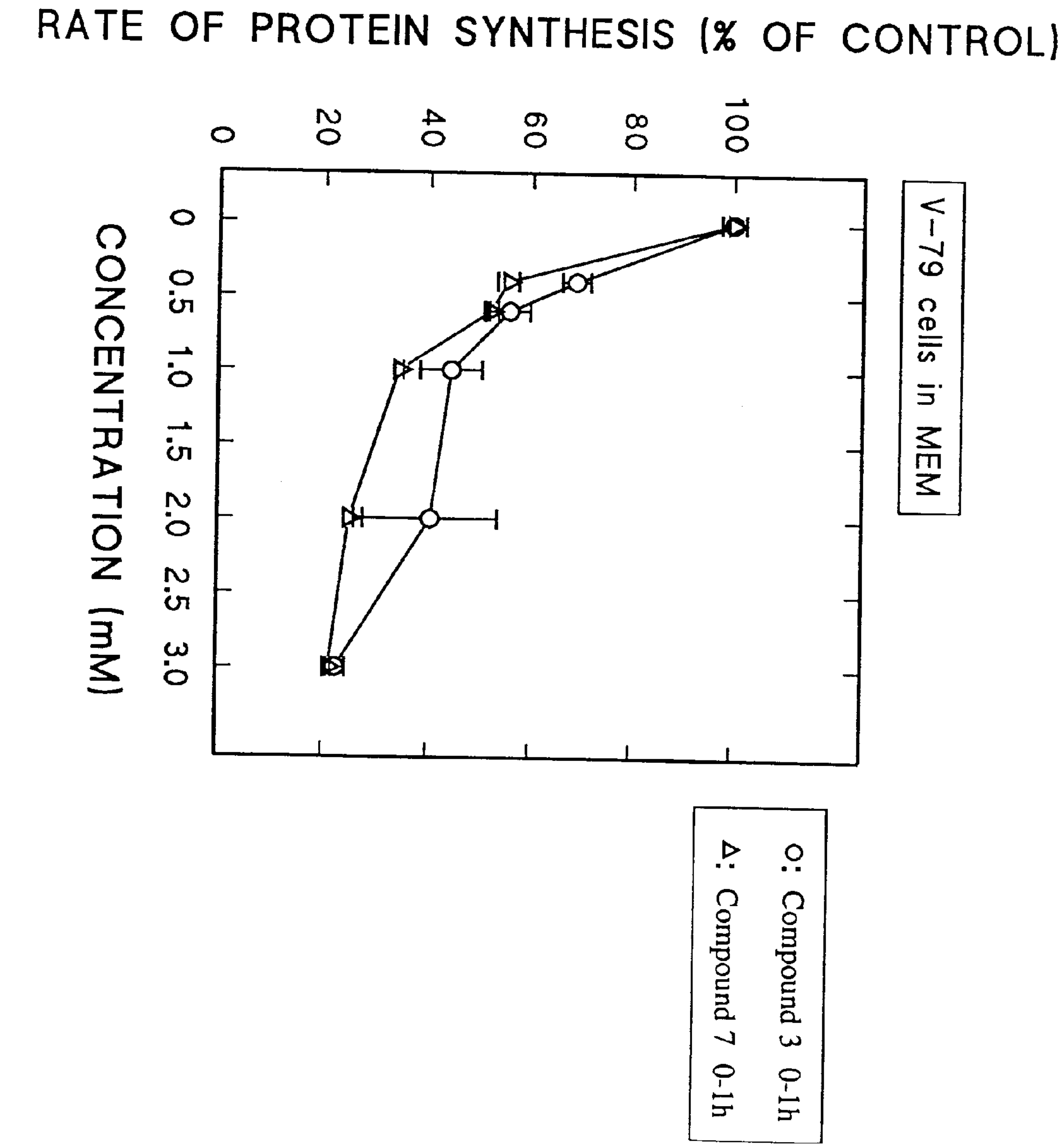
RATE OF PROTEIN SYNTHESIS (% OF CONTROL)
0
20
40
60
80
100
V-79 cells in MEM
0
0.5
1.0
1.5
2.0
2.5
3.0
CONCENTRATION (mM)
o: Compound 3 0-1h
Δ: Compound 7 0-1h

FIGURE 4

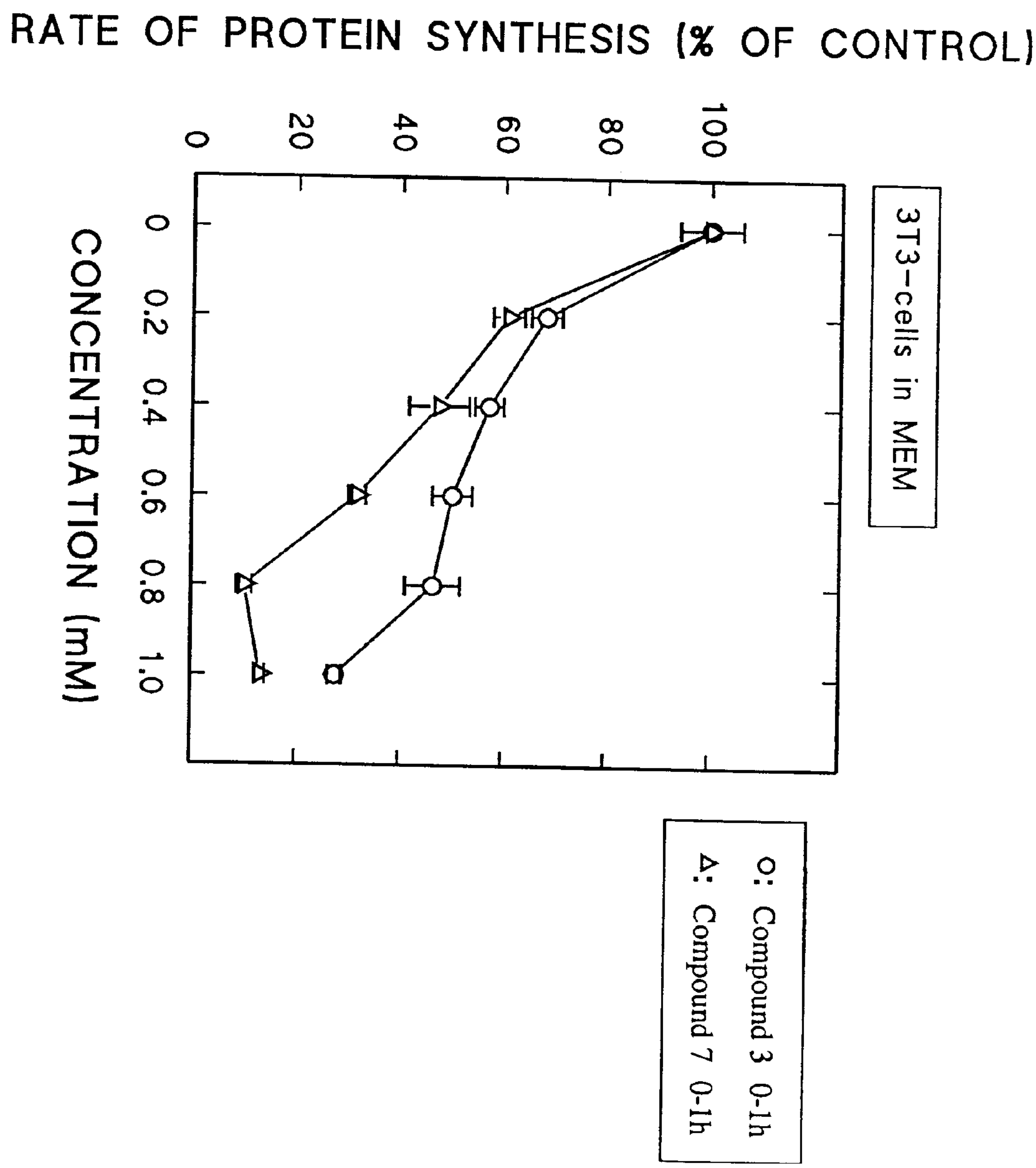
RATE OF PROTEIN SYNTHESIS (% OF CONTROL)
0
20
40
60
80
100
3T3-cells in MEM
0
0.2
0.4
0.6
0.8
1.0
CONCENTRATION (mM)
O: Compound 3 0-1h
Δ: Compound 7 0-1h

FIGURE 5

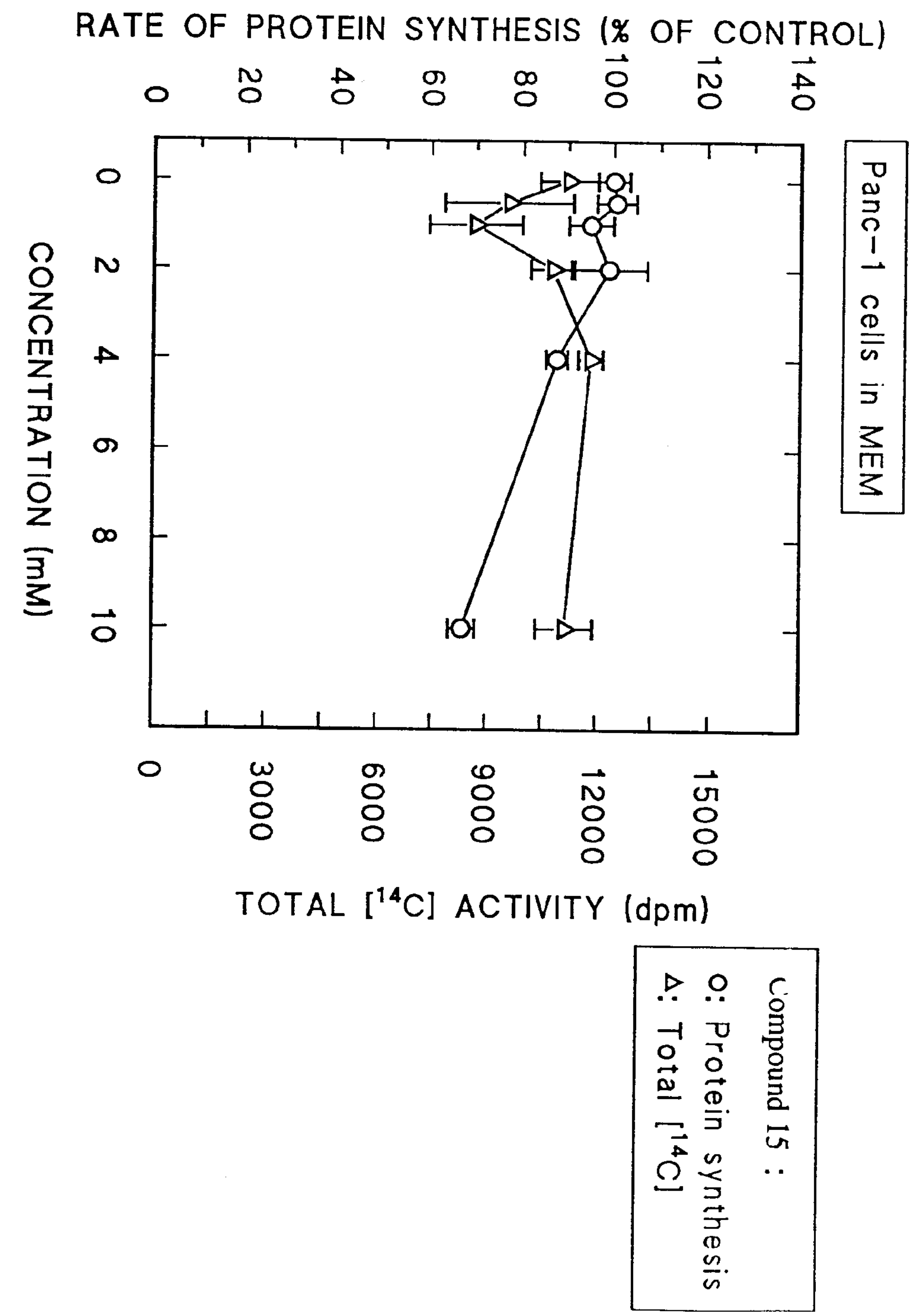
RATE OF PROTEIN SYNTHESIS (% OF CONTROL)
0
20
40
60
80
100
120
140
Panc-1 cells in MEM
0
2
4
6
8
10
CONCENTRATION (mM)
0
3000
6000
9000
12000
15000
TOTAL [14C] ACTIVITY (dpm)
Compound 15 :
o: Protein synthesis
Δ: Total [14C]

FIGURE 6 A

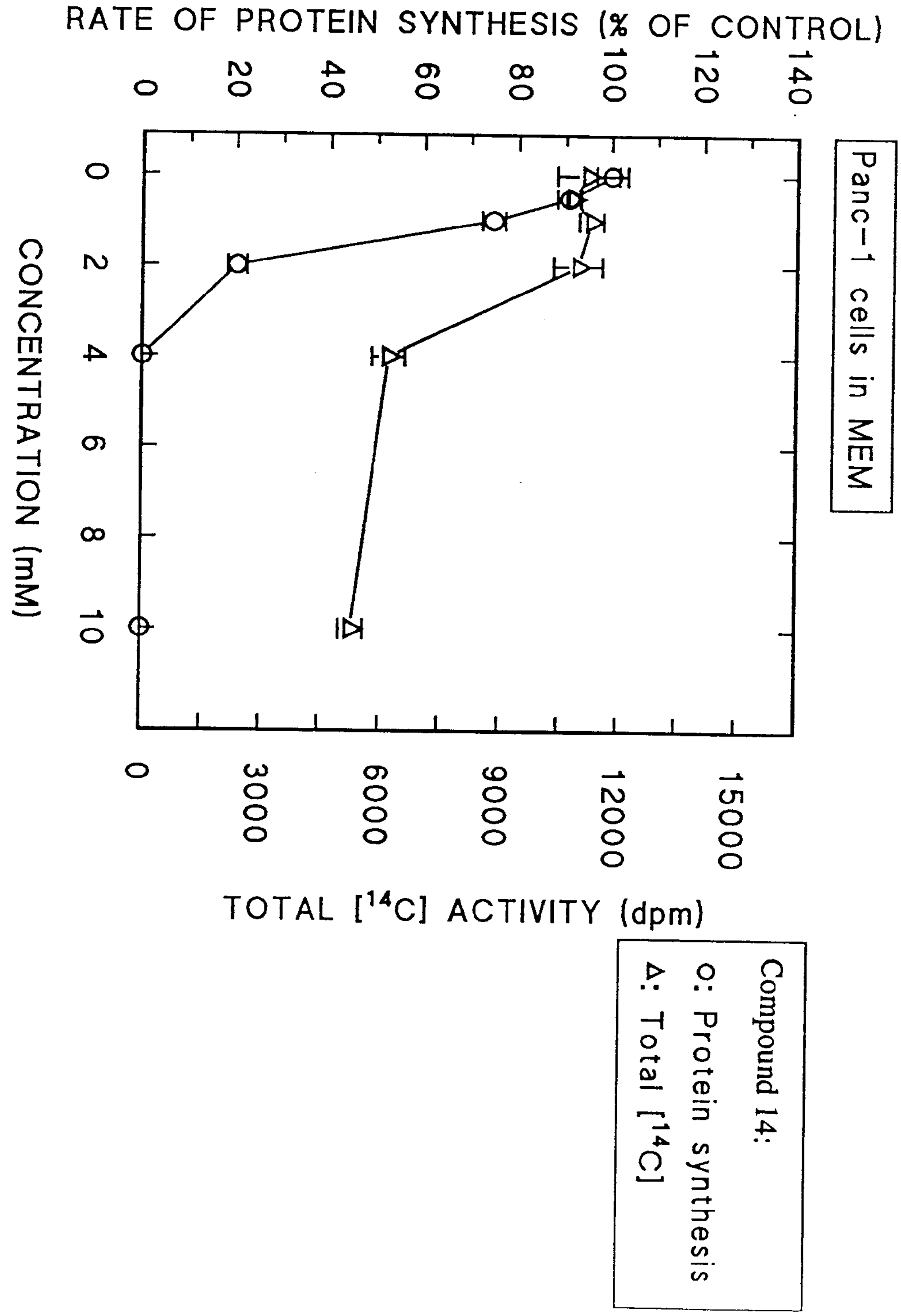

Panc–1 cells in MEM
RATE OF PROTEIN SYNTHESIS (% OF CONTROL)
0
20
40
60
80
100
120
140
CONCENTRATION (mM)
0
2
4
6
8
10
TOTAL [14C] ACTIVITY (dpm)
0
3000
6000
9000
12000
15000
Compound 14:
o: Protein synthesis
Δ: Total [14C]


FIGURE 6 B

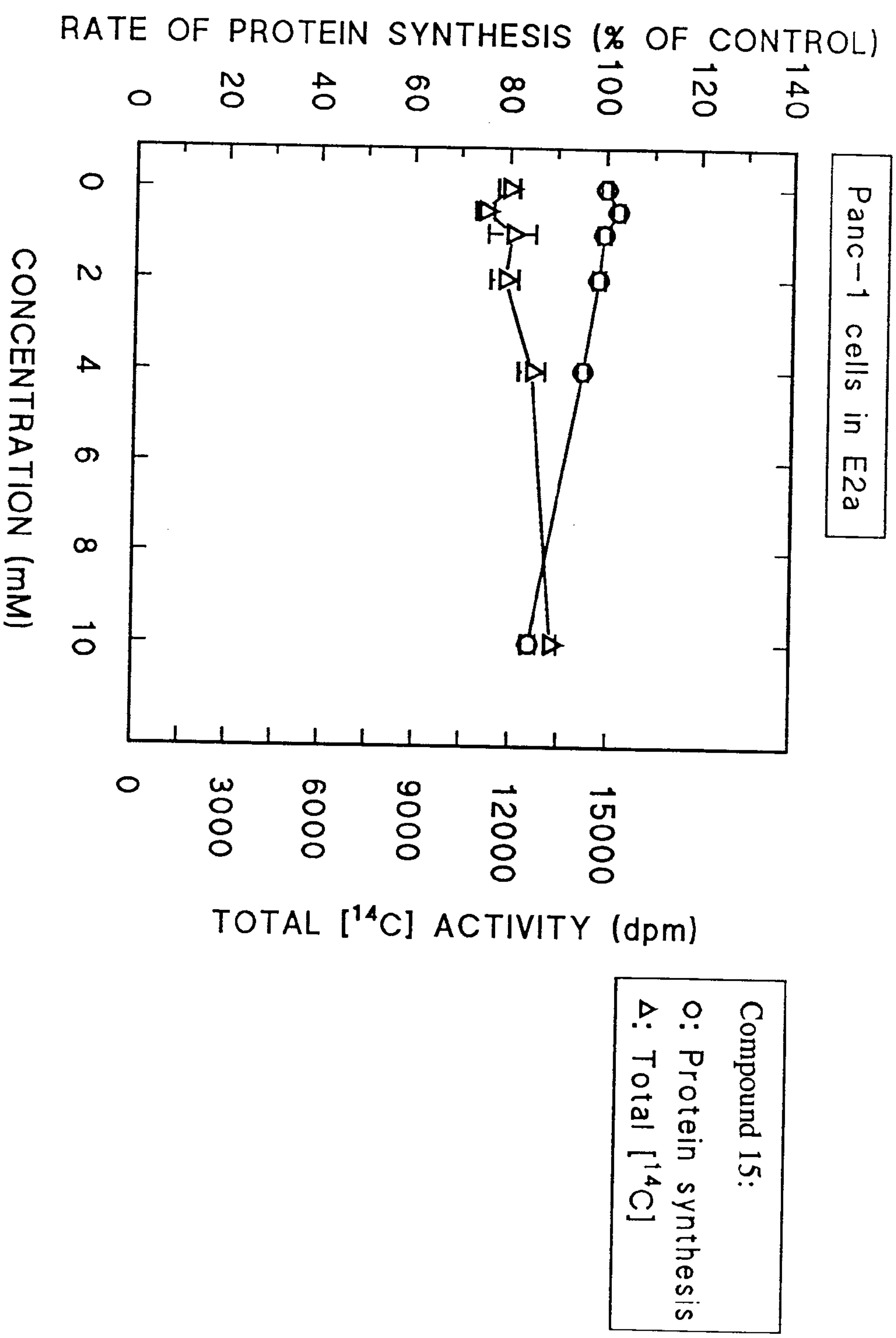
Panc−1 cells in E2a
RATE OF PROTEIN SYNTHESIS (% OF CONTROL)
0
20
40
60
80
100
120
140
0
2
4
6
8
10
CONCENTRATION (mM)
0
3000
6000
9000
12000
15000
TOTAL [14C] ACTIVITY (dpm)
Compound 15:
o: Protein synthesis
Δ: Total [14C]

FIGURE 7 A

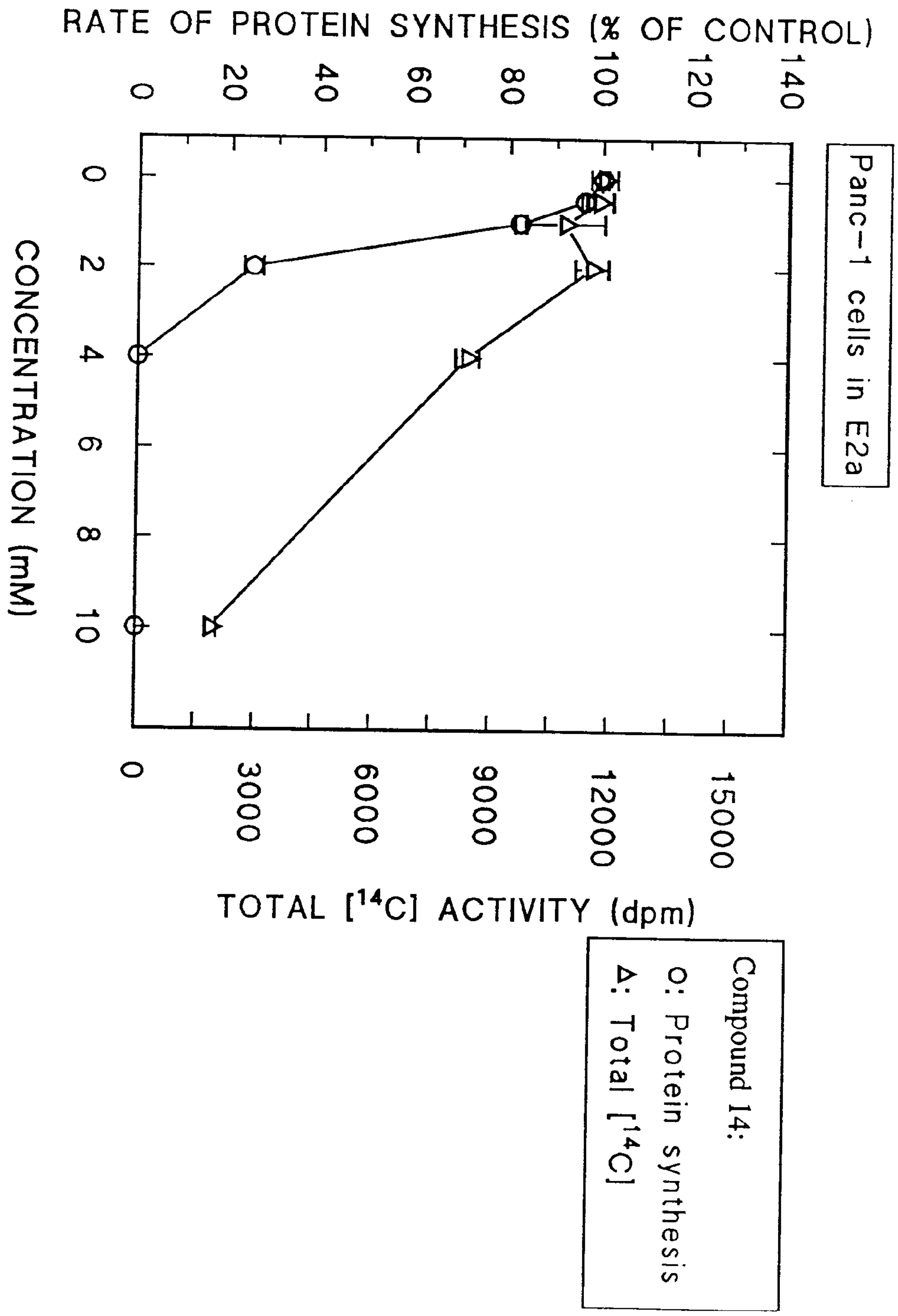
Panc-1 cells in E2a
RATE OF PROTEIN SYNTHESIS (% OF CONTROL)
0
20
40
60
80
100
120
140
CONCENTRATION (mM)
0
2
4
6
8
10
TOTAL [14C] ACTIVITY (dpm)
0
3000
6000
9000
12000
15000
Compound 14:
o: Protein synthesis
Δ: Total [14C]

FIGURE 7 B

European Patent Office

# EUROPEAN SEARCH REPORT

Application Number
EP 94 30 0454

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | PATENT ABSTRACTS OF JAPAN vol. 9, no. 324 (C-320) (2047) 19 December 1985 & JP-A-60 155 114 (RIKAGAKU KENKYUSHO) 15 August 1985 * abstract * --- | 1,3,5-9 | C07C251/24<br>A61K31/13<br>A61K31/195<br>A61K31/42<br>C07D263/04<br>C07D239/54<br>C07D407/04<br>A61K31/505 |
| X | PATENT ABSTRACTS OF JAPAN vol. 3, no. 147 (C-66) 5 December 1979 & JP-A-54 125 627 (RIKAGAKU KENKYUSHO) 29 September 1979 * abstract * --- | 1,3,5-9 | |
| X | PATENT ABSTRACTS OF JAPAN vol. 11, no. 60 (C-405) (2507) 24 February 1987 & JP-A-61 218 567 (RIKAGAKU KENKYUSHO) 29 September 1986 * abstract * --- | 1,3,5-9 | |
| X,D | PATENT ABSTRACTS OF JAPAN vol. 13, no. 72 (C-570) 17 February 1989 & JP-A-63 264 411 (RIKAGAKU KENKYUSHO ET AL.) 1 November 1988 * abstract * --- | 1,3,5-9 | **TECHNICAL FIELDS SEARCHED (Int.Cl.5)**<br>C07C<br>A61K<br>C07D |
| X | EP-A-0 041 827 (KUREHA KAGAKU KOGYO K.K.) * the whole document * | 1,3,5-9 | |
| Y | * abstract * --- | 1,3,5-9 | |
| Y | DE-A-34 14 049 (KUREHA KAGAKU KOGYO K.K.) * the whole document * --- | 1,3,5-9 | |
| X | DE-A-25 19 193 (LAWSON ALEXANDER) * page 10 - page 29 * --- | 1,3,5-7 | |
| | -/-- | | |

The present search report has been drawn up for all claims

| Place of search | Date of completion of the search | Examiner |
|---|---|---|
| BERLIN | 17 March 1994 | Rufet, J |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 94 30 0454

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | PATENT ABSTRACTS OF JAPAN vol. 10, no. 350 (C-387) 26 November 1986 & JP-A-61 151 161 (TOKUYAMA SODA LTD) 9 July 1986 * abstract * | 1,3 | |
| X | TETRAHEDRON, vol.41, no.24, 1985, OXFORD GB pages 5919 - 5928 MARIO E. ALVA ASTUDILLO ET AL. 'hydroxy schiff base-oxazolidine tautomerism...' * page 5920 * | 1,3 | |
| X | PATENT ABSTRACTS OF JAPAN vol. 9, no. 17 (C-262) (1740) 24 January 1985 & JP-A-59 164 759 (SUMITOMO KAGAKU KOGYO K.K.) 17 September 1984 * abstract * | 1,3 | |
| | | | TECHNICAL FIELDS SEARCHED (Int.Cl.5) |

The present search report has been drawn up for all claims

| Place of search | Date of completion of the search | Examiner |
|---|---|---|
| BERLIN | 17 March 1994 | Rufet, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)